# FASCICULE DE BREVET EUROPEEN

(11) **EP 0 584 328 B1**
(45) Date de publication et mention de la délivrance du brevet: **13.12.1995**
(21) Numéro de dépôt: 93905408.6
(22) Date de dépôt: 19.02.1993
(51) Int. Cl.: C07C 309/83, C07C 309/82, C07C 309/81, C07C 309/15, C07C 309/10, C07F 7/18, C07D 303/22, C08G 65/32

(54) **MONOMERES DERIVES DE SULTONES PERHALOGENEES ET POLYMERES OBTENUS A PARTIR DE CES MONOMERES**
VON PERHALOGENIERTEN SULTONEN ABGELEITETEN MONOMERE,SOWIE AUS DIESEN MONOMEREN HERGESTELLTE POLYMERE
PERHALOGENATED SULTONE-DERIVED MONOMERS AND POLYMERS PRODUCED THEREFROM

(30) Priorité: 21.02.1992 FR 9202027
(43) Date de publication de la demande: 02.03.1994
(73) Titulaire: CENTRE NATIONAL DE LA RECHERCHE SCIENTIFIQUE (CNRS), 75794 Paris Cédex 16 (FR); HYDRO-QUEBEC, Montréal Québec H2Z 1A4 (CA)
(72) Inventeur: ARMAND, Michel, F-38410 Saint-Martin-d'Uriage (FR); SANCHEZ, Jean-Yves, F-38330 Saint-Ismier (FR); SYLLA, Salime, Bamako (ML)
(74) Mandataire: Sueur, Yvette
(86) Numéro de dépôt international: FR9300167
(87) Numéro de publication internationale: WO9316988

(56) Documents cités:
- WO-A-92/02571
- DE-A- 2 461 675
- DE-A- 3 047 439
- US-A- 3 718 627

## Description

La présente invention concerne des monomères dérivés de sultones perhalogénées, leur procédé de préparation, les polymères obtenus à partir des dits monomères et leur utilisation pour l'élaboration de matériaux à conduction ionique.

On connaît des composés ROCF₂CF(CF₃)SO₂F dans lesquels R est un groupement alkyle éventuellement halogéné comportant une double liaison vinylique, une liaison acétylénique, un groupe époxy ou des groupements (CH₃-CH₂-O)₂P(O)-CH₂-CH₂- ou CH₃CH₂-O-P-F(O)-CH₂-CH₂, ainsi que leur procédé de préparation à partir d'une sultone fluorée cyclique (Chen et al, J. of Fluorine Chemistry, 48 (1990) 107-122) ; Chen et al, J. of Fluorine Chemistry, vol.46, 1990, p.21-38 ; Chen et al, J. of Fluorine Chemistry, vol.46, 1990, p.39-56). Des composés dans lesquels un groupe -SO₃M, M étant H, Li, Na, NH₄, K, est fixé sur un radical R¹R²-N-C(O)-CH-(CH₂)ₙ-COOM, n étant 0 ou 1, R¹ et R² étant des groupes alkyles, sont décrits dans US-A-4490308 (D.W. Fong et al). EP-A-0124378 (Du Pont De Nemours) décrit des monomères CH₂ = CH-CF₂-CF₂-OCF₂-CF₂-SO₂F, des copolymères de ces monomères avec de l'éthylène, l'utilisation de ces copolymères comme matériaux isolants électriques. Dans Chem. Abs., vol. 114, 1991, n°23718b, Huaxue, sont décrits des composés résultant de la réaction d'un fluorure de difluoroiodo-méthane sulfonyle avec une oléfine en présence de cuivre, par exemple nBu-CH = CH-CF₂-SO₂F, Me-C(CH₂)-C(CH₃)₂-CF₂ SO₂F ou CH₂ = CH-(CH₂)₂-CH = CH-CF₂ SO₂F.

Par le brevet européen N°13199 (Armand et Duclot), on connait les électrolytes polymères obtenus par dissolution d'un sel M⁺X⁻ dans un polymère solvatant comportant des hétéroatomes, M⁺ représentant H⁺, un cation métallique, un cation organique du type ammonium, amidinium ou guanidinium; X représentant un anion à charge électronique délocalisée, par exemple Br⁻, ClO₄⁻, R_{F}SO₃⁻ ou (R_{F}SO₂)₂N⁻ ; R_{F} représentant un groupement perfluoroalkyle ou perfluoroaryle. Ces électrolytes polymères ont de nombreuses applications, en particulier dans le domaine des générateurs électrochimiques, des systèmes de modulation de la lumière (M. Armand et al, EP-87401555), des capteurs, par exemple pour des membranes sélectives ou de référence (A. Hammou et al, FR-86.09602). De nombreux travaux ont été effectués, en particulier pour l'amélioration des propriétés de conduction de ces matériaux. Ils ont abouti par exemple à la formation de copolymères à base d'oxyde d'éthylène (M. Armand et al, FR-83.09886) ou de réseaux réticulés par des ponts uréthanes (H. Chéradame et al, FR-8007135, US-4,357,401). Ces matériaux ont cependant pour caractéristique commune de présenter une mobilité de l'anion X⁻ qui est, dans la plupart des cas, supérieure à celle du cation M⁺. Cette propriété est un inconvénient, en particulier pour les systèmes électrochimiques pour lesquels la réaction d'électrode fait intervenir le cation M⁺. Le passage du courant entraîne l'établissement d'un gradient de concentration du sel dissous qui augmente la résistivité de l'électrolyte et peut amener la formation à proximité des interfaces, de composés stoechiométriques polymère-sel présentant une conductivié ionique insuffisante.

Plusieurs tentatives ont été faites pour synthétiser des polymères contenant des groupements anioniques attachés à la chaîne macromoléculaire. Les ionophores contenant des groupements alkylsulfonates RSO₃⁻, des groupements carboxylates ou des groupements perfluorocarboxylates -R_{F}CO₂⁻ [D.J. Bannister et al, Polymer **25**, 1291 (1984) ; E. Tsushida et al, Macromolécules **21**, 96 (1988) ] sont cependant très peu dissociés dans les polymères solvatants de type polyéther comme le polyoxyde d'éthylène. Les conductivités ioniques obtenues avec de tels matériaux sont ainsi très faibles et ne permettent pas d'envisager d'applications pratiques.

Par ailleurs, des groupements perfluorosulfoniques -R_{F}SO₃⁻ constituent les groupements ionophores des membranes échangeuses d'ions de type NAFION®. De telles membranes, ainsi que les monomères à partir desquels elles sont obtenues, sont décrits par exemple dans DE-A-3047439 (Asahi Kasei Kogyo KK), DE-A-2461675 (Du Pont De Nemours), US-A-3718627 (W.G. Grot) ou dans US-A-3714245 (R. Beckerbauer). La partie réticulable des monomères ainsi que le squelette des polymères constituant les membranes sont perfluorés. Ces polymères ne présentent de conductivité ionique qu'en présence de solvants polaires tels que l'eau, les alcools, le carbonate de propylène ; la trame perfluorée de ce type de polymère n'a en effet aucun caractère solvatant ni dissociant, contrairement aux polyéthers.

Des polymères -(CX₂CXR)ₙ- ou -(CXRCXR)ₙ- dans lequels X est H ou F et R représente -(CX₂)ₘ-SO₃H, ainsi que leur utilisation comme électrolyte dans des piles à combustible sont décrits dans Chem. Abstr., vol. 117, 1992, n°27945h Matsushita.

WO-A-9202571 (SRI International) décrit des polymères à conduction ionique portant des anions greffés -X-Y⁻M⁺, X pouvant être CF₂ ou CFR_{F}, Y pouvant être SO₃ et M⁺ étant un cation. Le squelette des polymères est dérivé d'un polymère linéaire ayant des hydrogènes mobiles. Il peut s'agir par exemple d'un squelette du type polyéther, polyester, poly(éthylène)imine, polyphosphazène, siloxane. Les polymères peuvent être obtenus soit en greffant les groupes appropriés sur n'importe quelle chaîne polymérique ayant des hydrogènes mobiles, soit en polymérisant des monomères à cycle oxazoline portant un groupe -SO₂F.

La présente invention a pour but de fournir des monomères permettant d'obtenir des polymères particulièrement intéressants pour l'élaboration de matériaux à conduction cationique.

C'est ainsi que la présente invention a pour objet des monomères perhalogénés.

Elle a également pour objet un procédé de préparation de ces monomères.

Elle a en outre pour objet les polymères obtenus à partir des dits monomères.

Enfin, elle a pour objet des matériaux à conduction ionique contenant les dits polymères.
Les monomères de la présente invention sont des composés répondant à la formule générale (1) : 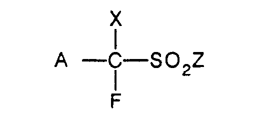
dans laquelle:
- A représente l'un des groupes R³-O-CF₂-, ou R³- ou 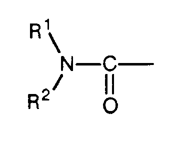
- Z représente F, Cl, -OSi(CH₃)₃ ou un groupement ionique ; Z étant différent de F lorsque A représente R³-O-CF₂- ou R³- ;
- X représente F, Cl, H ou R_{F} ; X étant R_{F} lorsque A représente R³- ;
- les radicaux R¹, R² et R³, identiques ou différents, sont choisis parmi les radicaux organiques non perfluorés polymérisables ;
- R_{F} est choisi parmi les radicaux perfluoroalkyle et les radicaux perfluoroaryle.

Les groupements ioniques Z particulièrement préférés sont choisis parmi 1/mM^{m+}[-O]⁻, 1/mM^{m+}[-N-SO₂-Q]⁻, 1/mM^{m+}[-CH(SO₂-Q)]⁻ et 1/mM^{m+}[-C(SO₂-Q)₂]⁻, Q représentant -R_{F} ou -CFX-A, avec X = R_{F} lorsque A = R³ ; M^{m+} représentant un ion d'un métal ayant la valence m, choisi parmi les métaux alcalins, les métaux alcalino-terreux, les métaux de transition et les terres rares, ou un ion ammonium répondant à la formule NH₍₄₋ⱼ₎Rⱼ⁺, ou un ion amidinium répondant à la formule RC(NH₂₋ⱼRⱼ)₂⁺, ou un ion guanidinium répondant à la formule C(NH₂₋ⱼRⱼ)₃⁺, avec j = 0, 1 ou 2, R étant choisi parmi l'hydrogène, un groupement alkyle, oxaalkyle ou aryle.

Parmi les différents cations M^{m+}, ceux qui correspondent à des réactions d'électrodes, par exemple dans les systèmes de stockage d'énergie, de modulation du rayonnement électromagnétique, les électrodes de référence, sont particulièrement intéressants. A cet égard, les ions Li⁺, Na⁺, K⁺, Cs⁺, NH₄⁺, Ca⁺⁺, Cu⁺⁺, Zn⁺⁺, Mg⁺⁺ sont particulièrement préférés.

Parmi les groupements R_{F} du type perfluoroalkyle, on préfère les radicaux perfluoroalkyle ayant de 1 à 8 atomes de carbone, et plus particulièrement les radicaux perfluoroalkyle ayant de 1 à 4 atomes de carbone. On peut citer les radicaux CF₃-, C₂F₅-, n-C₃F₇-, n-C₄F₉-.

Parmi les groupements R_{F} du type perfluoroaryle, on préfère les radicaux perfluoroaryle ayant de 6 à 8 atomes de carbone, par exemple le radical perfluorophényle.

Les groupements organiques non perfluorés polymérisables R¹, R² et R³ permettent des réactions de polymérisation par voie radicalaire, anionique, cationique ou stéréospécifique, ou par polycondensation. Ils peuvent être choisis parmi ceux qui comportent des doubles liaisons, par exemple des doubles liaisons du type vinyle, allyle, vinylbenzyle ou acryloyle. Ils peuvent également être choisis parmi ceux qui comportent des fonctions oxirane, oxétane, azétidine ou aziridine. En outre, ils peuvent être choisis parmi ceux qui comportent des fonctions alcool, thiol, amine, isocyanate ou trialcoxysilane. Ils peuvent également être choisis parmi ceux qui comportent des fonctions permettant une électropolymérisation.

Le substituant R des groupements ammonium, amidinium ou guanidinium est choisi de préférence parmi H, les groupements alkyles ayant de 1 à 20 atomes de carbone, les groupement oxalkyle ayant de 1 à 20 atomes de carbone ou les groupements aryle ayant de 6 à 30 atomes de carbone. On préfèrera tout particulièrement les groupements méthyle, éthyle, propyle, lauryle et méthoxyéthyle.

Parmi les monomères de la présente invention, on préfère tout particulièrement ceux qui répondent à la formule générale (1), dans laquelle Z représente un groupement ionique. A titre d'exemple, on peut citer les familles suivantes :
* 1/mM^{m+}[A-CFX-SO₃]⁻,
* 1/mM^{m+}[A-CFX-SO₂-N-SO₂-R_{F}]⁻, 1/mM^{m+}[A-CFX-SO₂-CH(SO₂-R_{F})]⁻, 1/mM^{m+}[A-CFX-SO₂-C(SO₂-R_{F})₂]⁻,
* 1/mM^{m+}[A¹-CFX¹-SO₂-N-SO₂-CFX²-A²]⁻, 1/mM^{m+}[A¹-CFX¹-SO₂-CH(SO₂-CFX²-A²)]⁻, 1/mM^{m+}[A¹-CFX¹-SO₂-C(SO₂-CFX²-A²)₂]⁻,
A¹ et A², identiques ou différents, étant choisis parmi les groupements A cités précédemment ; X¹ et X², identiques ou différents, étant choisis parmi les groupements X cités précédemment, étant entendu que X est R_{F} si A correspondant est R³.

Les composés monomères suivants sont particulièrement intéressants : (C₃H₅)₂NCOCF₂SO₂F, K[(C₃H₅)₂NCOCF₂SO₃], Na[(C₃H₅)₂NCOCF₂SO₃], Li[(C₃H₅)₂NCOCF₂SO₃], (C₃H₅)₂NCOCF(CF₃)SO₂F, K[(C₃H₅)₂NCOCF(CF₃)SO₃], Na[(C₃H₅)₂NCOCF(CF₃)SO₃], Li[(C₃H₅)₂NCOCF(CF₃)SO₃], Li[H₂C=CHCH₂O(CF₂)₂SO₃], Li[(C₃H₅)₂NCOCF(CF₃)SO₂]₂N, Li[H₂C=CHCH₂O(CF₂)₂SO₂(CF₃SO₂)₂C], K[CH₂OHCHOHCH₂O(CF₂)₂SO₃], Li[(CH₃O)₃Si(CH₂)₃N(CH₃)COCF(CF₃)SO₃], K[H₂C=CHCOCF(CF₃)SO₃],

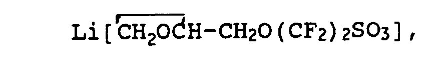

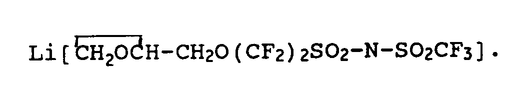

Les composés de la présente invention peuvent tous être obtenus avantageusement à partir d'une sultone cyclique (2) obtenue directement par réaction entre le trioxyde de soufre SO₃ et un dérivé polyhalogéné de l'éthylène, et transformée quantitativement en fluorure d'acide sulfonylacétique (3) par isomérisation en présence d'une base nucléophile. La base nucléophile catalysant l'isomérisation de la sultone cyclique peut être choisie parmi les éthers (par exemple le diéthyléther, le tétrahydrofurane, les glymes), les fluorures ioniques, les bases azotées (par exemple les pyridines, les trialkylamines). 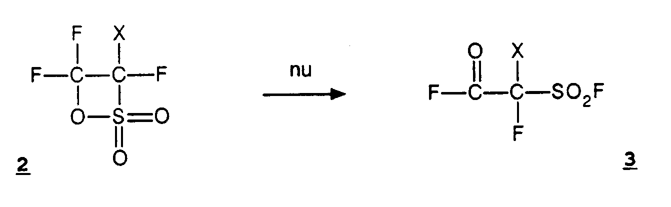
Les composés de la présente invention répondant à la formule (1) dans laquelle A représente R¹R²N-CO- et Z représente F, désignés ci-après par composés (4), sont obtenus par réaction du fluorure d'acide sulfonylacétique (3) sur une amine en présence d'une base, selon le schéma réactionnel suivant: 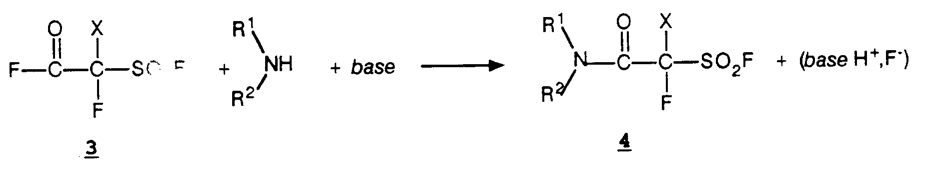
Dans un mode de réalisation préféré, la base permettant la neutralisation de l'acide HF formé au cours de la réaction est une résine échangeuse d'ion de type faiblement basique, par exemple une amine tertiaire, ce qui permet la séparation facile par simple filtration des sous-produits de la réaction. Dans un autre mode de réalisation, l'amine R¹R²NH en excès peut être utilisée comme base.

Un composé de la présente invention répondant à la formule (1) dans laquelle A représente R¹R²N-CO- et Z est différent de F, désigné ci-après par composé (5), est obtenu en faisant réagir le composé (4) avec le réactif approprié.
Ainsi, un composé (5a) dans lequel Z = 1/mM^{m+}[-O⁻] est obtenu par réaction d'un composé (4) avec 1/mM^{m+}OH⁻ ou avec 1/mM^{m+}[OSi(CH₃)₃]⁻.
Un composé (5b) dans lequel Z est Cl est obtenu par réaction d'un composé (4) avec un chlorure 1/mM^{m+}Cl⁻, de préférence avec un chlorure alcalin ou alcalino-terreux, suivant la réaction :

A-CFX-SO₂F + 1/mM^{m+}Cl⁻ ---> A-CXF-SO₂Cl + 1/mM^{m+}F⁻.

Un compose (5c) dans lequel Z est -OSi(CH₃)₃ est obtenu par réaction d'un composé (4) avec un agent de silylation, en particulier l'hexaméthyldisiloxane selon la réaction :

A-CFX-SO₂F + (CH₃)₃SiOSi(CH₃)₃ --> A-CFX-SO₃Si(CH₃)₃ + FSi(CH₃)₃

Un composé (5d) pour lequel Z = 1/mM^{m+}[-NSO₂Q]⁻ est obtenu par réaction du composé (4) correspondant avec 1/mM^{m+}[HNSO₂Q]⁻ en présence d'une base nucléophile.
Un composé (5e) pour lequel Z = 1/mM^{m+}[-CH(SO₂Q)]⁻ est obtenu par réaction du composé (4) correspondant avec 1/mM^{m+}[H₂C(SO₂Q)]⁻ en présence d'une base nucléophile.
Un composé (5f) pour lequel Z = 1/mM^{m+}[-C(SO₂Q)₂]⁻ est obtenu par réaction du composé (4) correspondant avec 1/mM^{m+}[HC(SO₂Q)₂]⁻ en présence d'une base nucléophile.
Dans les composés (5d) à (5f), Q représente A-CFX- ou R_{F}-.
La base nucléophile est choisie de préférence parmi les trialkylamines [par exemple la triéthylamine, la diisopropyléthylamine, la quinuclidine, le 1,4 diazabicyclo[2,2,2]octane (TED)], les pyridines (par exemple la pyridine, les alkylpyridines, les dialkylaminopyridines), les imidazoles (par exemple les N-alkylimidazoles, l'imidazo [1,2-a]pyridine), les amidines, par exemple le 1,5-diazabicyclo[4,3,0]non-5-ène (DBN) et le 1,8 diazabicyclo [5,4,0] undec-7-ène (DBU)], les guanidines [par exemple la tétra méthylguanidine et la 1,3,4,7,8 hexahydro-1-méthyl-2H-pyrimido[1,2-a]-pyrimidine (HPP)].

Les composés de la présente invention répondant à la formule (1) dans laquelle A représente R³O-CF₂-, désignés ci-après par famille (6), sont obtenus à partir du fluorure d'acide sulfonylacétique (3) par un procédé en trois étapes. Au cours d'une première étape, on fait réagir le fluorure d'acide sulfonylacétique (3) avec un fluorure M'F ; au cours d'une seconde étape, on met en contact le perfluoroalcoxyde (7) obtenu avec un réactif R³Y pour obtenir le composé R³O-CF-CFX-SO₂F (8) suivant les schémas réactionnels : 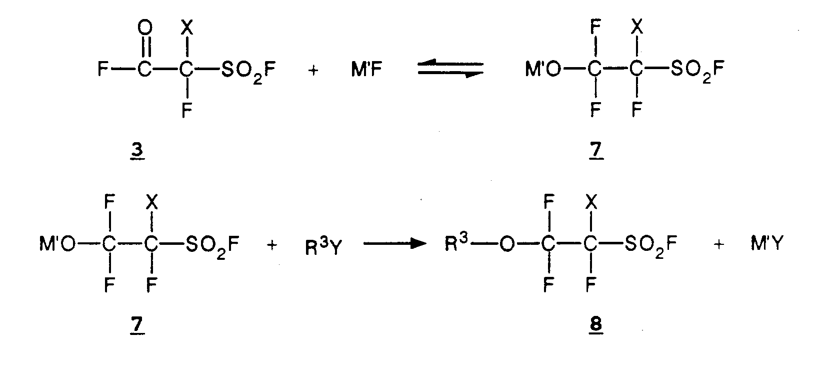
Enfin, au cours d'une troisième étape, on fait réagir le composé (8) avec le réactif approprié pour remplacer le fluor du groupe SO₂F par Cl, -OSi(CH₃)₃, un groupement ionique choisi parmi 1/mM^{m+}[-O]⁻, 1/mM^{m+}[-NSO₂R]⁻, 1/mM^{m+}[-CH(SO₂R_{F})]⁻ ou 1/mM^{m+}[-C(SO₂R_{F})₂]⁻, suivant des procédés analogues à ceux décrits pour l'obtention des composés (5) à partir d'un composé (4).
Le fluorure M'F est un fluorure d'un cation métallique ou d'un cation organique peu polarisant. Parmi les cations appropriés, on peut citer les ions Ag⁺, K⁺, Rb⁺, Cs⁺, les ions tétraalkyl-ammonium, les ions tétraalkyl-phosphonium et les ions tetra(dialkylamino)phosphonium.
Dans le réactif R³Y, Y représente un groupe partant tel que Cl, Br, I, un alkyl-sulfonate, un aryl-sulfonate ou un perfluoroalkylsulfonate.

Les composés de la présente invention répondant à la formule (1) dans laquelle A représente R³-, Z est différent de F et X représente R_{F}, désignés ci-après par composés (9), sont obtenus à partir du fluorure d'acide sulfonylacétique (3) par un procédé en trois étapes. Au cours d'une première étape, le fluorure d'acide sulfonylacétique est traité par l'eau, ce qui provoque une hydrolyse suivie d'une décarboxylation selon le schéma réactionnel suivant : 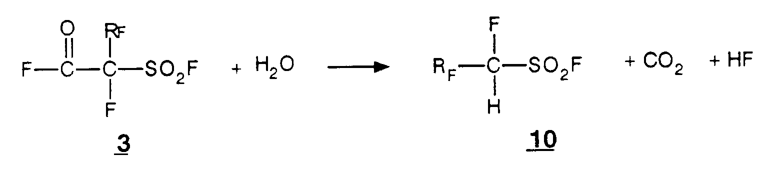
Le composé (10) obtenu possède sur le carbone en α un proton présentant un caractère acide permettant la formation d'un carbanion qui donne lieu, au cours d'une deuxième étape, à une réaction de substitution nucléophile en présence d'une base, qui permet d'obtenir le composé (11) selon le schéma réactionnel suivant : 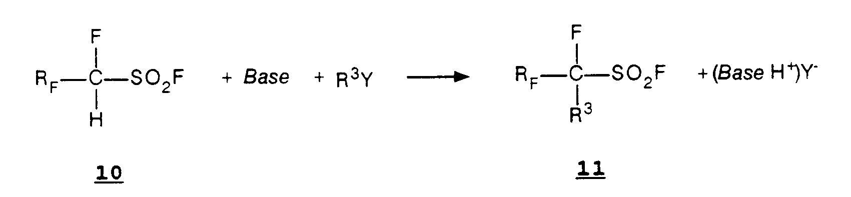
La base est choisie de préférence parmi les dérivés azotés présentant un encombrement stérique empêchant la formation d'un ammonium quaternaire par réaction directe sur R³Y.
Au cours d'une troisième étape, on fait réagir le composé (11) obtenu avec le réactif approprié pour remplacer le fluor du groupe SO₂F par Cl, -OSi(CH₃)₃, un groupement ionique choisi parmi 1/mM^{m+}[-O]⁻, 1/mM^{m+}[-NSO₂R_{F})]⁻, 1/mM^{m+}[-CH(SO₂R_{F})]⁻ ou 1/mM^{m+}[-C(SO₂R_{F})₂]⁻, selon des procédés analogues à ceux décrits pour l'obtention des composés (5) à partir d'un composé (4).

Lorsqu'un composé monomère de la présente invention est un composé symétrique tel que 1/mM^{m+}[(A-CFX-SO₂)₂N]⁻, 1/mM^{m+}[(A-CFX-SO₂)₂CH]⁻ ou 1/mM^{m+}[(A-CFX-SO₂)₃C]⁻, il peut être préparé par action directe du fluorure d'acide sulfonylacétique A-CFX-SO₂F, obtenu par isomérisation de la sultone cyclique correspondante, sur le nitrure ionique Li₃N ou le carbure ionique C₃Al₄ dans un solvant aprotique polaire, le groupement A représentant R¹R²N-CO- ou R³O-CF₂- ou R³-.

Bien entendu, les monomères de la présente invention dans lesquels Z représente un groupement ionique obtenus par l'un des procédés décrits ci-dessus peuvent être modifiés par les procédés classiques d'échange de cations, par exemple à l'aide de résines échangeuses d'ions, en vue de remplacer le cation M par un autre cation.

Lorsque les groupes polymérisables existant dans les groupements R¹, R² et R³ présentent des fonctions dont la réactivité pourrait interférer avec les réactions de préparation des monomères telles que décrites ci-dessus, les dites fonctions peuvent être protégées par des réactifs qui leurs sont liés d'une manière réversible.

Les composés monomères de la présente invention peuvent être polymérisés grâce aux fonctions polymérisables présentes dans les groupements R¹, R² et R³. Les polymères obtenus selon la présente invention portent des fonctions -SO₂Z.

Selon une première variante, on obtient un polymère linéaire dont la trame est constituée par les groupements R¹, R² ou R³ polymérisés et porte des greffons présentant des terminaisons -SO₂Z. Ce polymère est obtenu par homopolymérisation d'un composé monomère de la présente invention. Les composés monomères de la présente invention peuvent être homopolymérisés lorsque le groupement A comporte au moins deux fonctions réactives complémentaires. A titre d'exemple, on peut citer les groupements A comportant un groupe diallyle amino, vinylphénylamino ou acrylamidoéthyl amino.

Selon une deuxième variante, les polymères de la présente invention sont obtenus sous forme de copolymères à partir d'au moins un monomère de la présente invention et d'un ou plusieurs comonomères. Au moins l'un des comonomères est choisi de telle sorte qu'il comporte une fonction capable de réagir avec le groupement polymérisable du monomère selon l'invention. Les comonomères peuvent en outre être choisis en fonction des propriétés particulières qu'ils conféreront au copolymère. A cet égard, on peut également introduire dans le copolymère, des monomères additionnels ne réagissant pas avec le monomère de l'invention, mais intéressants par leurs propriétés.

Les copolymères peuvent être obtenus par deux voies différentes. Suivant une première voie, on obtient un polymère contenant des unités monomères de la présente invention en coréticulant les composes monomères de la présente invention avec un homopolymère ou un copolymère préexistant comportant des fonctions capables de réagir avec la fonction portée par l'un au moins des groupements R¹, R² ou R³. Les réseaux macromoléculaires ainsi obtenus sont constitués par une trame macromoléculaire constituée par l'homopolymère ou le copolymère préexistant sur laquelle sont greffées les unités monomères selon l'invention, comportant un groupe -SO₂Z. Comme exemple de polymères préexistants, on peut citer les polymères obtenus par polymérisation d'allylglycidyléther, éventuellement en présence d'autres monomères tels que l'oxyde d'éthylène ou le méthyl glycidyléther, les copolymères butadiène-acrylonitrile, un copolymère de diisocyanate de α,ω diaminooligooxyéthylène et de poly(oxyéthylène-triol). Suivant une deuxième voie, les composés monomères de la présente invention peuvent être incorporés dans des polymères par copolymérisation directe avec un ou plusieurs co-monomères dont l'un au moins porte des fonctions capables de réagir avec la fonction portée par l'un au moins des groupements R¹, R² ou R³. Parmi ces monomères capables de polymériser avec un monomère de l'invention, on peut citer l'allylglycidyl éther, les époxyalcènes, les acrylates de glycidyle; parmi les monomères non réactifs on peut citer l'oxyde d'éthylène, l'oxyde de propylène, le méthylglycidyl éther.

Dans tous les cas, lorsque le monomère selon l'invention choisi est du type symétrique, c'est-à-dire lorsque le substituant Z comporte un groupement A-CFX-, qui peut être identique ou différent du groupement A-CFX- provenant du fluorure de l'acide acétylsulfonique de départ, les copolymères obtenus sont réticulés.

Lorsque les polymères de la présente invention sont obtenus à partir de monomères selon l'invention comportant un groupement Z ionique, ils peuvent être traités de manière classique, par exemple à l'aide de résines échangeuses de cations, pour remplacer un cation M^{m+} par un cation différent.

Les polymères et les réseaux macromoléculaires de la présente invention sont utiles pour l'élaboration de matériaux à conduction ionique.

Les polymères ou les réseaux macromoléculaires incorporant des monomères selon la présente invention sont particulièrement intéressants lorsque Z représente un groupement ionique choisi parmi 1/mM^{m+}[-O]⁻, 1/mM^{m+}[-NSO₂Q]⁻, 1/mM^{m+}[-CH(SO₂Q)]⁻ et 1/mM^{m+}[-C(SO₂Q)₂]⁻, Q représentant R_{F} ou [R¹R²NCOCF(X)-] ou [R³OCF₂CF(X)-] ou [R³CF(R_{F})-]. Dans ce cas, ils portent des anions greffés sur la chaîne macromoléculaire et constituent en eux-même des matériaux à conduction ionique sans adjonction supplémentaire de sels. La présence de sels facilement dissociables peut toutefois être utile.

Lorsque les polymères et les réseaux macromoléculaires de la présente invention portent un substituant Z qui n'est pas un groupement ionique, il est nécessaire d'y ajouter un sel facilement dissociable lors de l'élaboration d'un matériau à conduction ionique.

Les matériaux à conduction ionique de l'invention, et plus spécialement ceux dans lesquels les anions sont fixés sur la chaîne macomoléculaire, sont particulièrement utiles dans les systèmes électrochimiques pour lesquels la réaction d'électrode fait intervenir les cations, par exemple dans les cellules électrochimiques comportant une anode contenant du lithium. Parmi ces systèmes électrochimiques, on peut citer les générateurs électrochimiques au lithium, les systèmes électrochromes, ou encore les capteurs comportant des membranes sélectives ou des membranes de référence.

La présente invention est décrite plus en détail dans les exemples suivants, donnés à titre illustratif, mais non limitatif.

### EXEMPLE 1

La perfluropropanesultone (12) est obtenue par addition de l'hexafluoroprène sur le trioxyde de soufre et le composé est purifié par distillation (T_{eb} = 45°C sous 10⁵ Pa).

Dans un ballon muni de quatre cols permettant de recevoir une ampoule à brome, une entrée et une sortie d'argon sec et un palier d'agitation mécanique, on introduit 50 ml d'éther éthylique anhydre et 4 g de résine échangeuse d'ions faiblement basique (Amberlyst® A-21) préalablement séchée sous vide à 60°C. Le réacteur est refroidi à -20°C et on ajoute goutte à goutte 4,6 g d'hexafluoropropanesultone. L'isomérisation exothermique est catalysée par la résine basique, donnant le fluorure d'acide FCOCF(CF₃)SO₂F. L'agitation est continuée pendant dix minutes après la fin de l'addition de la sultone.

1,9 g de diallylamine sont alors ajoutés très lentement à la solution précédente de manière à être toujours en défaut par rapport à la sultone isomérisée et permettre seulement l'attaque de la fonction la plus réactive, le fluorure d'acide carboxylique (-COF). Après la fin de l'addition de la diallylamine, l'agitation est maintenue pendant 30 min tandis que le mélange réactionnel revient à température ambiante. Aprés filtration de la solution pour séparer la résine, l'éther est évaporé et le liquide obtenu est distillé sous pression réduite sur une colonne à bande tournante. Le produit final (13) est un liquide incolore, soluble dans de nombreux solvants organiques tels que l'éther, l'acétone, le THF, l'acétonitrile et le pentane.

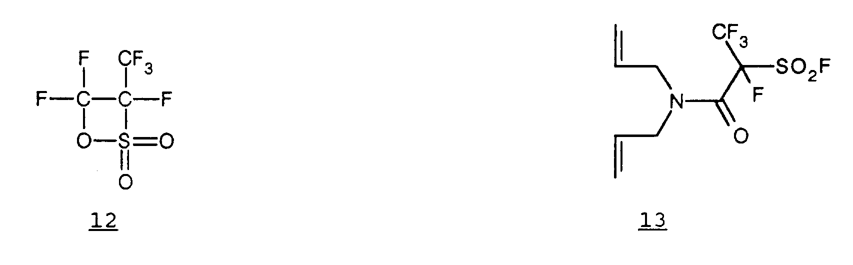

### EXEMPLE 2

1,5 g du fluorure de sulfonyle (13) obtenu à l'exemple 1 ont été dissous dans 5 ml de THF et transformés en sel alcalin par action de 636 mg de triméthylsilanoate de potassium dissous dans 10 ml de THF. La réaction a été rapide et totale à température ambiante avec élimination de fluorotriméthylsilane gazeux (Eb = 16°C) dans les conditions de l'expérience.

(C₃H₅)₂NC0CF(CF₃)SO₂F + (CH₃)₃SiOK --- > K[(C₃H₅)₂NC0CF(CF₃)SO₃]+ (CH₃)₃SiF

Le THF a été chassé, le résidu repris à l'éther et le sel récupéré après filtration et évaporation de l'éther. 1,42 g de sel de potassium ont ainsi été obtenus (rendement >90%). Il se présente sous forme de poudre blanche, peu hygroscopique, soluble dans l'eau et dans de nombreux solvants organiques tels que le THF et l'acétonitrile.

Le sel de sodium peut être préparé de la même manière à partir du triméthylsilanoate de sodium.

### EXEMPLE 3

A 2 g du fluorure de sulfonyle obtenu à l'exemple 1 dissous dans 10 ml de THF sont ajoutés 600 mg de monohydrate de lithine. La suspension est agitée à température ambiante pendant 48 heures. La disparition totale du fluorure de sulfonyle est vérifiée par chromatographie en phase vapeur (CPV). La réaction se déroule selon le schéma :

(C₃H₅)₂NC0CF(CF₃)SO₂F + 2 LiOH,H₂O ---> Li[(C₃H₅)₂NC0CF(CF₃)SO₃] + LiF + 3H₂O

Le mélange est ensuite centrifugé puis, après évaporation du solvant, le résidu est repris dans l'éther et centrifugé ; le processus est répété 3 fois. Le sel est finalement précipité dans le pentane et séché à 60°C sous vide pendant 48h avant d'être conservé sous argon.

### EXEMPLE 4

6,6 g d'un ter-polymère d'oxyde d'éthylène, de méthylglycidyl éther et d'allyl-glycidyl éther de masse M̅_̅{̅w̅}̅=2,5.10⁵ sont mis en solution dans l'acétonitrile. A la solution ainsi obtenue, on ajoute 1% en poids par rapport au polymère de peroxyde de benzoyle et 2,3 g du monomère ionique de l'exemple 3. Un film de complexe polymère-sel d'une épaisseur de 40 »m est obtenu par coulage de la solution dans un anneau de verre posé sur une plaque de poly(tétrafluoroéthylène) et évaporation du solvant. Le complexe peut être schématisé par la formule suivante : 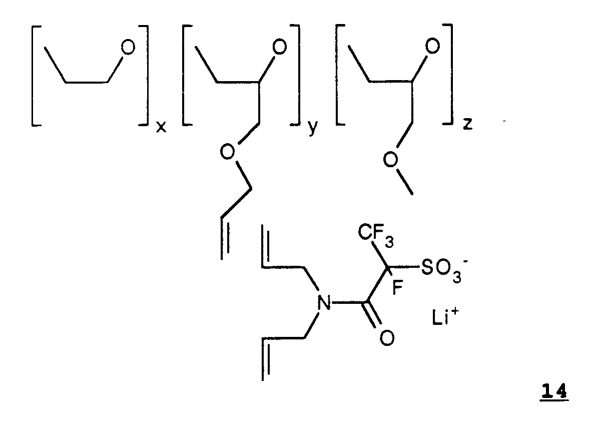
Le film est ensuite chauffé sous atmosphère d'argon sec à 80°C pendant 3 h. Les doubles liaisons des groupements allyle du polymère et celles du sel ajouté réagissent entre elles après amorçage par le peroxyde pour former une trame réticulée. Le film obtenu est lavé plusieurs fois à l'acétone pour éliminer le monomère résiduel et les oligomères de faible masse moléculaire. La Résonance Magnétique Nucléaire confirme l'immobilité des anions (mesure par la méthode du gradient de champ pulsé sur le ¹⁹F du sel). Le film a d'excellentes propriétés mécaniques et sa conductivité est de 7.10⁻⁵ (Ωcm)⁻¹ à 65°C. Sa structure peut être schématisée par :

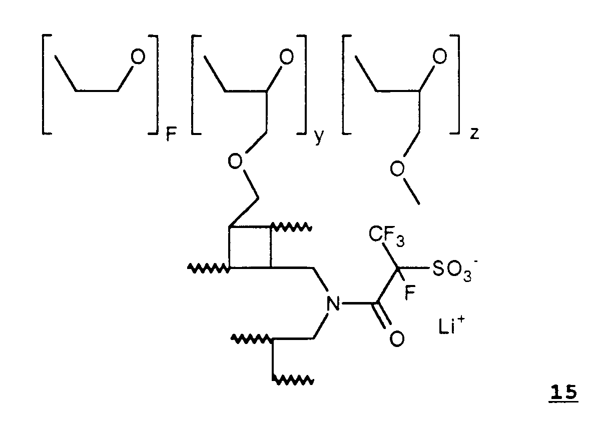

### EXEMPLE 5

11 g de méthacrylate de méthoxy(oligooxyéthylène) formé par estérification du méthoxy(polyéthylène glycol) de masse 1000 par le chlorure de méthacryloyle sont mélangés à 50 ml d'eau et la solution est déoxygénée par barbotage d'argon et maintenue à 5°C. Ensuite 200 mg de persulfate de potassium K₂S₂O₈ associé à 260 mg de sulfate ferreux FeSO₄,7H₂O sont ajoutés en maintenant le mélange sous agitation, de même que 3,1 g du monomère ionique de l'exemple 2. La polymérisation est arrêtée par addition de 200 mg d'hydroquinone. Le mélange réactionnel est purifié par ultrafiltration, permettant l'élimination des molécules (sels, monomères, oligomères) de masses moléculaires inférieures à 10K daltons. Ensuite, le copolymère est séché sous vide à 60°C pendant plusieurs jours. L'étude de son spectre IR permet de calculer la quantité de sel incorporé dans le copolymère. La conductivité du copolymère est de 1.10⁻⁵ (Ωcm)⁻¹ à 53°C.

### EXEMPLE 6

On a élaboré un générateur électrochimique secondaire comportant des polymères selon la présente invention. L'anode est constituée par un film de lithium métallique de 14 »m d'épaisseur laminé sur une feuille de polypropylène de 8 »m, métallisé par 100 nm de nickel. L'électrolyte est le copolymère (15) de l'exemple 4. L'électrode positive est un matériau composite contenant 45% en volume de manganite de lithium de structure spinelle LiMn₂O₄, broyé en grain de ≅ 8 »m, 5% v/v de noir de carbone de type Ketjenblack EC-600-JD et 50% v/v du polymère de l'exemple 5. Un mélangeur à galets est utilisé pour obtenir une dispersion homogène des constituants de l'électrode positive dans l'acétonitrile, permettant, par une technique d'épandage, d'obtenir des films de 60 »m d'épaisseur sur un collecteur de courant constitué par une feuille de polypropylène métallisée par du nickel, similaire à celui de l'électrode négative. Le laminage des composants permet d'obtenir un générateur souple de 131 »m d'épaisseur, caractérisé par une f.e.m. de 3V, un débit de 500 »A/cm² à 70°C pour une tension de 2,8 V. Ce système est rechargeable.

### EXEMPLE 7

Du fluorure de glycidyloxytétrafluoroéthanesulfonyle de formule : 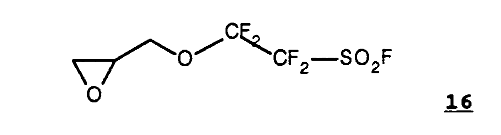
est préparé selon la méthode de Gard et al. [J. Fluorine Chemistry **46** 21 (1990) ; ibidem **46** 39(1990) ; ibidem **48** 107(1990)] par action de l'épibromhydrine sur le produit d'addition du fluorure d'argent AgF sur la tétrafluoroéthanesultone isomérisée dans le diglyme. Après élimination du bromure d'argent formé, le produit est distillé (Eb : 115°C sous 1,2.10⁴ Pa).
2,59 g de fluorure de glycidyloxytétrafluoroéthanesulfonyle (16) sont transformés en sel de potassium correspondant : 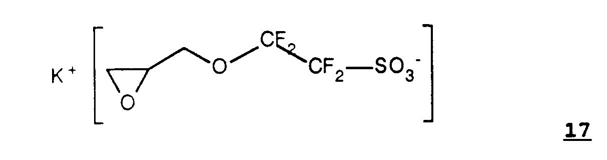
par action d'une quantité stoechiométrique de triméthylsilanoate de potassium dans le THF. Le sel est purifié par recristallisation dans l'eau.

Un réacteur connecté à une rampe à vide est chargé avec 440 mg de tertiobutoxyde de potassium et 2,9 g du sel de potassium (17). Le système est dégazé sous vide et on admet dans le réacteur 25 g de THF, 13,5 g d'oxyde d'éthylène et 1,15 g d'allylglycidylether distillés sur miroirs de sodium. La polymérisation est effectuée à 80°C pendant 8 heures. Le copolymère est précipité dans l'hexane et purifié par dissolution dans 50 ml d'acétone et précipitation dans l'éther éthylique. Cette opération est répétée six fois ; dans les trois premières opérations, 4 g de trifluorométhane sulfonate de lithium sont dissous dans l'acétone, permettant un échange des ions potassium du polymère par les ions lithium. Le polymère obtenu est séché sous vide. Sa conductivité à 25°C est de 2,2 10⁻⁶ (Ωcm)⁻¹. Ce polymère contient les unités : 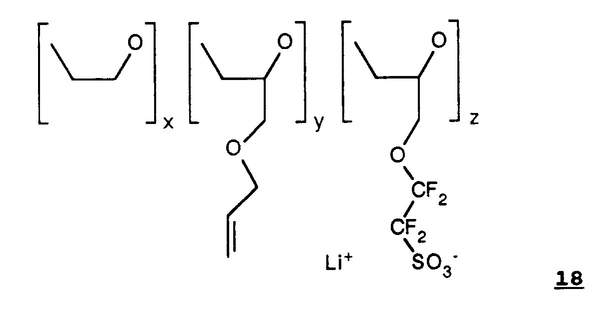
x ≅ 30, y ≅ 1 ; z ≅ 1
Un film réticulé ayant de bonnes propriétés mécaniques peut être obtenu par addition de 0,5% de peroxyde de benzoyle et chauffage à 80°C sous atmosphère d'argon pendant 24 heures. La conductivité ionique de ce matériau est de 1.10⁻⁵ (Ωcm)⁻¹ à 41°C.

### EXEMPLE 8

Le fluorure d'allyloxytétrafluroéthanesulfonyle de formule : 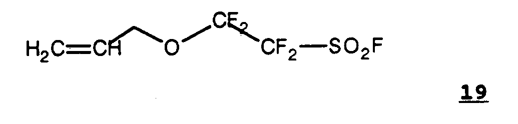
est préparé par une méthode analogue à celle de l'exemple 7 en remplaçant l'épibromhydrine par le bromure d'allyle. Le produit est purifié par distillation (Eb :121°C sous 2,2.10⁴ Pa). Le dérivé correspondant du lithium est obtenu par action de l'hydroxyde de lithium dans le méthanol, filtration et séchage.
4 g d'un copolymère d'oxyde d'éthylène (90%) et d'allylglycidyléther (10%) sont dissous dans 30 ml d'acétonitrile et on ajoute 800 mg du sel de lithium tel que préparé et 30 mg de peroxyde de benzoyle. La solution est coulée pour former après évaporation un film de 40 »m d'épaisseur. Ce matériau est chauffé à 80°C sous atmosphère d'argon pendant 8 heures, ce qui permet une co-réticulation du sel et du polymère qui possédent des doubles liaisons réactives dont l'ouverture est initiée par les radicaux libres issus de la décomposition du peroxyde de benzoyle. Le réseau polymère obtenu est lavé dans quatre bains successifs d'acétone pour éliminer le sel n'ayant pas réagi et les traces d'oligomères. Le film, après séchage, a une conductivité de 10⁻⁵ (Ωcm)⁻¹ à 40°C.

### EXEMPLE 9

6,1 g du fluorure de sulfonyle préparé à l'exemple 1 sont dilués dans 30 ml de THF anhydre, auquel on ajoute 350 mg de nitrure de lithium Li₃N. Le mélange est maintenu sous agitation pendant 48 heures à la température ordinaire. Après filtration et évaporation du solvant, on obtient 5,5 g du sel de lithium de la sulfonimide : 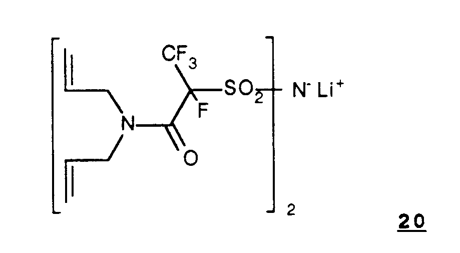
Ce sel est incorporé par co-réticulation à un polymère ionique semblable à celui de l'exemple 4. Un réseau contenant 17% en poids de sel fixé (20) a une conductivité de 3.10⁻⁵ (Ωcm)⁻¹ à 35°C.

### EXEMPLE 10

2,4 g de fluorure d'allyloxytétrafluroéthanesulfonyle préparé selon l'exemple 8 sont mis en solution dans un mélange d'acétonitrile (20 ml) et de pyridine (10 ml) anhydres, et la solution est traitée par 3,02 g de sel de sodium du bis(trifluorométhanesulfonyle)méthane auquel sont ajoutés 150 »l de triéthylamine et 50 mg de diméthylaminopyridine, suivant le schéma réactionnel :

Na(CF₃SO₂)₂CH + QSO₂F + (C₂H₅)₃N ---> NaF + [QSO₃(CF₃SO₂)₂C]⁻,(C₂H₅)₃NH⁺

Q représentant ici le groupement allyloxytétrafluroéthane.

Le dérivé correspondant du lithium est obtenu par action de 1,2 g de phosphate lithium en suspension dans le méthanol, filtration et séchage.

D'une manière similaire à celle de l'exemple 8,4 g d'un copolymère d'oxyde d'éthylène (90%) et d'allylglycidyléther (10%) sont dissous dans 30 ml d'acétonitrile et on ajoute 1,2 g du sel de lithium tel que préparé et 40 mg de peroxyde de benzoyle. La solution est coulée pour former après évaporation un film de 35 »m d'épaisseur. Ce matériau est chauffé à 80°C sous atmosphère d'argon pendant 8 heures, pour obtenir une co-réticulation du sel et du polymère. Le réseau polymère obtenu est lavé dans quatre bains successifs d'acétone pour éliminer le sel n'ayant pas réagi et les traces d'oligomères. Le film, après séchage, a une conductivité de 3.10⁻⁵ (Ωcm)⁻¹ à 40°C.

### EXEMPLE 11

2,59 g de fluorure de glycidyloxytétrafluoroéthanesulfonyle de l'exemple 7 sont transformés en sel de potassium de l'acide 2,2-dihydroxypropyloxytétrafluoroéthanesulfonique par traitement par 22 ml d'une solution de potasse 1 M à 80°C pendant 1 heure. Le pH est ajusté à 7 à l'aide d'une solution d'acide sulfurique 1M et l'eau est chassée à l'aide d'un évaporateur rotatif. Le solide obtenu est extrait avec 25 ml d'éthanol anhydre, le sulfate de potassium insoluble est éliminé par filtration. Après séchage, on obtient 2,4 g du sel de structure : 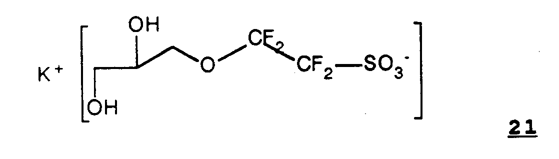
12,7 g de α,ω diaminooligooxyéthylene (Jeffamine® ED600, commercialisée par Texaco) sont traités par 1,96 g de bis(trichlorométhylcarbonate) (triphosgène) en présence de 5 g de résine basique Amberlyst® A-21, commercialisé par Rohm & Haas, pour former le diisocyanate correspondant ; 6,8 g de ce composé, 8,8 g de poly(oxyéthylène triol) de masse 2000 (commercialisé par Denko Kagaku), 926 mg du sel (21) et 40 mg de 4-diméthylaminopyridine sont mélangés dans 10 ml de dichlorométhane et coulés entre deux plaques de verre séparées par un joint de 1 mm d'épaisseur. La polycondensation s'effectue en 48 heures, catalysée par la diméthylaminopyridine. La membrane est démoulée et extraite par de l'acétone pour éliminer le sel n'ayant pas réagi et les traces d'oligomères. L'opération est répétée quatre fois. La membrane, après séchage, a une épaisseur de 400 »m, et une conductivité de 9.10⁻⁶ (Ωcm)⁻¹ à 25°C. Cette membrane a d'excellentes propriétés mécaniques et peut être utilisée dans des dispositifs à transmission optique modulable, utilisant en particulier le Bleu de Prusse K[Fe₂(CN)₆] comme matériau électrochrome.

### EXEMPLE 12

2,44 g du sel de sodium de l'exemple 10 sont mis en solution dans 10 ml d'eau et traités par 2,7 mg de bromure de tétrapropylammonium. Le précipité d'allyloxytétrafluroéthanesulfonate de tétrapropylammonium est recristallisé dans l'eau chaude.

5 g de copolymère de butadiène (70 moles %) et d'acrylonitrile (30 moles %) sont dissous dans l'acétone. A la solution homogène sont ajoutés 800 mg du sel d'ammonium préparé précédemment et 100 mg de peroxyde de benzoyle. La solution est coulée pour former après évaporation un film de 35 »m d'épaisseur. Ce matériau est chauffé à 80°C sous atmosphère d'argon pendant 8 heures, pour obtenir une coréticulation du sel et du polymère. On obtient un matériau dont les propriétés mécaniques sont comparables à celles de l'élastomère butadiène-acrylonitrile sans adjonction d'alpyloxytétrafluroéthanesulfonate de tétrapropylammonium. Le matériau incluant le monomère de l'invention a une conductivité de 3.10⁻⁷ (Ωcm)⁻¹ à la température ambiante et de bonnes propriétes antistatiques.

### EXEMPLE 13

A 2,27 g de N-méthyl-aminopropyl triméthoxysilane en solution dans 15 ml d'éther éthylique anhydre maintenu à 0°C, sont ajoutés 2,3 g de phosphate de lithium Li₃PO₄ anhydre. Par une ampoule à brome sont ajoutés goutte à goutte 2,3 g de perfluoropropane sultone, puis 1 ml de pyridine anhydre. Le mélange est agité sous argon pendant 24 heures en laissant la température remonter à l'ambiante. La suspension est filtrée et évaporée. On obtient le sel de lithium : 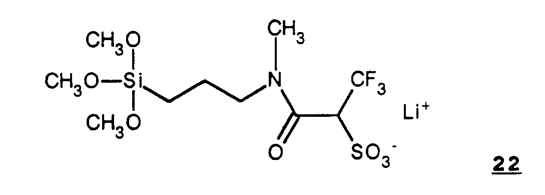
1,5 g d'isocyanatopropyltriméthoxysilane sont dissous dans 10 ml de THF anhydre et on ajoute 2,33 g de O,O'-bis (2-aminopropyl)polyéthylène glycol 800 (Jeffamine® ED 900 commercialisé par Texaco). Après réaction des liaisons isocyanate sur les groupements -NH₂ terminaux de l'oligomère (24 heures), formant des liaisons urée, on ajoute 400 mg du sel de lithium préparé précédemment qui se dissout ; le THF est éliminé dans un évaporateur rotatif. Un copolymère conducteur ionique est obtenu par hydrolyse-condensation de ce mélange de monomères. Pour cela, on ajoute 10 ml de méthanol contenant 0,5 ml d'eau. Ensuite, la solution est coulée dans un moule en PTFE et la polycondensation est effectuée dans une étuve à 60°C pendant 12 jours. On obtient ainsi un matériau transparent et flexible ayant cependant une grande résistance aux rayures. Sa conductivité ionique est de 1,5.10⁻⁶ (Ωcm)⁻¹ à 25°C. Ce polymère organosilicié peut être dissous dans l'eau et déposé en couche mince sur du verre, auquel il présente une bonne adhérence, pour constituer un vitrage électrochrome.

### EXEMPLE 14

23 g de perfluoropropylène sultone isomérisée de l'exemple 1 sont additionnés goutte à goutte à 100 ml d'eau maintenue à 1°C. Un dégagement de dioxyde de carbone accompagne la réaction. Le mélange diphasique est amené à un pH d'environ 5 par addition de bicarbonate de sodium. Après séparation de la phase aqueuse surnageante, le composé obtenu est distillé et l'on obtient 16 g de FC(CF₃)HSO₂F. 9,2 g de ce composé sont dissous dans 60 ml d'éther éthylique anhydre auxquels sont ajoutés 5 ml de pyridine et 5 ml de triéthylamine. 4,5 ml de chlorure d'acryloyle sont additionnés goutte à goutte. Le précipité de chlorhydrate de pyridinium formé est éliminé par centrifugation et l'éther est chassé à l'aide d'un évaporateur rotatif. Le composé obtenu est distillé, puis traité par une quantité stoechiométrique de triméthylsilanoate de lithium dans le THF. La structure du monomère-sel ainsi obtenu est représentée par: 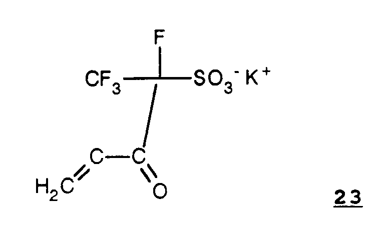
Ce composé possède une double liaison active suceptible d'homo-polymériser ou de co-polymériser pour former des polyélectrolytes.

### EXEMPLE 15

Un copolymère solvatant est préparé par condensation de glyoxal et de O,O'-bis(2-aminopropyl)polyéthylène glycol 500 (Jeffamine® ED600) en présence d'acide acétique. La polybase de Schiff ainsi obtenue est hydrogénée par le complexe hydrure de bore-triméthylamine pour donner un polymère séquencé contenant les séquences :

(C₂H₄O)≅₁₁[C₂H₄N(H)C₂H₄N(H)](C₂H₄O)≅₁₁.

Le polymère est purifié par ultrafiltration (coupure à 5K daltons). Sa structure est schématisée par 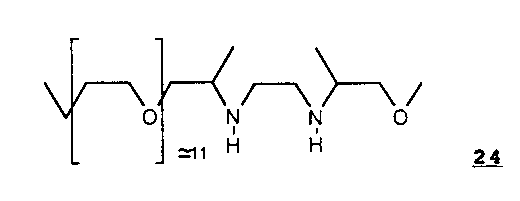
A 2 g de résine échangeuse d'ions faiblement basique (Amberlyst® A-21), préalablement séchée sous vide à 60°C, en suspension dans 40 ml d'acétonitrile anhydre maintenu à - 10°C, sont ajoutés goutte à goutte 2,3 g d'hexafluoropropanesultone. Après isomérisation, on ajoute par une ampoule à brome, 10 g du polymère séché sous vide à 80°C dissous dans 60 ml d'acétonitrile anhydre, puis 1,2 g de triméthylsilanoate de lithium en solution dans le THF. La solution obtenue est filtrée et le polymère est précipité par l'éther, puis purifié par 3 dissolutions successives dans l'acétone, suivies de précipitations dans l'éther. Le matériau obtenu peut être représenté par la structure suivante : 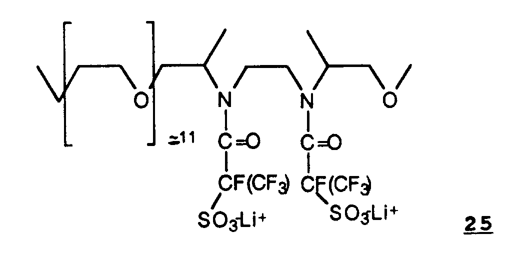
La conductivité ionique du polymère obtenu est de 1,5.10⁻⁵(Ωcm)⁻¹ à 25°C. Il est soluble dans l'eau et dans les solvants polaires aprotiques tels que l'acétonitrile, l'acétone, la diméthylformamide.

### EXEMPLE 16

La chlorotrifluoroéthane sultone est préparée par action du chlorotrifluoroéthylène sur l'anhydride sulfurique. On obtient après isomérisation le fluorure d'acide FCOC(Cl)FSO₂F qui est traité par la diallylamine dans les conditions de l'exemple 1. Par action de l'hydroxyde de lithium dans l'éthanol, on obtient Li[(C₃H₅)₂NC0CF(Cl)SO₃]. Un poly(méthoxy-éthoxy-éthoxyphosphazène) est préparé selon une variante du procédé décrit par H. R. Allcock, et al, Macromolecules 19, 1508 (1986), en remplaçant 5% en moles des groupements méthoxy-éthoxy éthanol par l'alcool allylique lors de la réaction de substitution des atomes de chlore du poly (dichlorophosphazène). Ce polymère-solvant contient alors des groupements solvatants et des motifs allyle permettant une réticulation. Le polymère obtenu peut être représenté par : 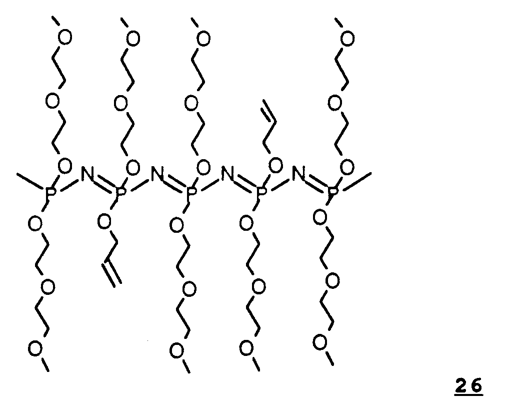
5 g de ce polymère, 800 mg du sel de lithium préparé précédemment et 150 mg de peroxyde de benzoyle sont co-dissous dans l'acétone et le solvant évaporé pour laisser un film de 37 »m d'épaisseur. Après traitement à 80°C sous argon pendant 2 h, on obtient une membrane élastique ayant une conductivité de 1,5.10⁻⁵(Ωcm)⁻¹ à 30°C.

### EXEMPLE 17

3,5 g du sel de l'exemple 3 sont dissous dans 30 ml d'eau et l'on ajoute 100 mg d'acide azobis (cyanovalérique). La solution est dégazée par barbotage d'argon et chauffée à 80°C pendant 4 h. Le polymère est précipité par le dioxane et séché sous vide primaire. Un monomère de type N-oligooxyéthylène-pyrrole est préparé par réaction de Jeffamine® M-100 sur le diméthoxytétrahydrofurane en présence d'acide acétique. Dans une cellule électrochimique contenant 50 ml d'acétonitrile, on dissout 900 mg du polymère ionique préparé ci-dessus et 1 g du pyrrole substitué. Une polymérisation par oxydation anodique est effectuée à + 0,95 V par rapport à l'électrode de référence Hg/Hg₂Cl₂ correspondant au passage de 220 Cb. L'électrode de travail se recouvre d'un film flexible de couleur noire contenant le polypyrrole dopé sous forme cationique, les charges étant compensées par celles des anions du polymère ionique. Ce matériau possède une conductivité mixte, ionique et électronique, et des propriétés électrochromes.

## Revendications

1. Composé répondant à la formule générale (1) suivante 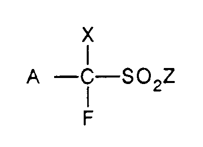 dans laquelle :
- A représente l'un des groupes R³-O-CF₂-, R³- ou 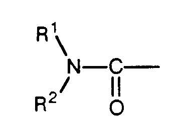
- Z représente F, Cl, -OSi(CH₃)₃ ou un groupement ionique; Z étant différent de F lorsque A représente R³-O-CF₂- ou R³-;
- X représente F, Cl, H ou R_{F} ; X étant R_{F} lorsque A représente R³-;
- les radicaux R¹, R² et R³ identiques ou différents, sont choisis parmi les radicaux organiques non perfluorés polymérisables;
- R_{F} est choisi parmi les radicaux perfluoroalkyle et les radicaux perfluoroaryle.

2. Composé selon la revendication 1, caractérisé en ce que Z représente un groupement ionique choisi parmi 1/mM^{m+}[-O]⁻, 1/mM^{m+}[-NSO₂Q]⁻, 1/mM^{m+}[-CH(SO₂Q)]⁻ et 1/mM^{m+}[-C(SO₂Q)₂]⁻, Q représentant -R_{F} ou -CFX-A, avec X = R_{F} lorsque A = R³ ; M^{m+} représentant un ion d'un métal ayant la valence m, choisi parmi les métaux alcalins, les métaux alcalino-terreux, les métaux de transition et les terres rares, ou un ion ammonium répondant à la formule NH₍₄₋ⱼ₎Rⱼ⁺, ou un ion amidinium répondant à la formule RC(NH₂₋ⱼRⱼ)₂⁺, ou un ion guanidinium répondant à la formule C(NH₂₋ⱼRⱼ)₃⁺, avec j = 0, 1 ou 2, R étant choisi parmi l'hydrogène, un groupement alkyle, oxaalkyle ou aryle.

3. Composé selon l'une quelconque des revendications 1 ou 2, caractérisé en ce que R_{F} est choisi parmi les groupements perfluoroalkyle ayant de 1 à 8 atomes de carbone, et les groupements perfluoroaryle ayant de 6 à 8 atomes de carbone.

4. Composé selon la revendication 1, caractérisé en ce que les groupements R¹, R² ou R³ comportent des doubles liaisons du type vinyle, allyle, vinylbenzyle ou acryloyle, ou une fonction oxirane, une fonction oxétane, une fonction azétidine, une fonction aziridine, une fonction alcool, une fonction thiol, une fonction amine, une fonction isocyanate ou une fonction trialcoxysilane.

5. Composé selon la revendication 1, caractérisé en ce qu'il répond à l'une des formules suivantes :
1/mM^{m+}[A-CFX-SO₃]⁻,
1/mM^{m+}[A-CFX-SO₂-N-SO₂-R_{F}]⁻,
1/mM^{m+}[A-CFX-SO₂-C(SO₂-R_{F})₂]⁻,
1/mM^{m+}[A-CFX-SO₂-CH(SO₂-R_{F})]⁻
1/mM^{m+}[A¹-CFX¹-SO₂-N-SO₂-CFX²-A²]⁻,
1/mM^{m+}[A¹-CFX¹-SO₂-CH(SO₂-CFX²-A²)]⁻,
1/mM^{m+}[A¹-CFX¹-SO₂-C(SO₂-CFX²-A²)₂]⁻,
dans lesquelles A, X et M ont la signification donnée précédemment ; A¹ et A², identiques ou différents, étant choisis parmi les groupements A cités précédemment ; X¹ et X², identiques ou différents, étant choisis parmi les groupements X cités précédemment, X étant R_{F} si A est R³.

6. Procédé de préparation d'un composé selon la revendication 1 dans lequel A représente R¹R²N-CO- et Z représente F, caractérisé en ce qu'il consiste à faire réagir le fluorure d'acide sulfonyl acétique F-COCFX-SO₂F avec une amine R¹R²NH en présence d'une base.

7. Procédé de préparation d'un composé selon la revendication 1 dans lequel Z représente Cl, caractérisé en ce qu'il consiste à faire réagir le composé A-CFX-SO₂F avec un chlorure 1/mM^{m+}Cl⁻.

8. Procédé de préparation d'un composé selon la revendication 1 dans lequel Z représente -OSi(CH₃)₃, caractérisé en ce qu'il consiste à faire réagir le composé A-CFX-SO₂F avec un agent de silylation tel que l'hexaméthyldisiloxane.

9. Procédé de préparation d'un composé selon la revendication 1 dans lequel Z représente 1/mM^{m+}[-O]⁻, caractérisé en ce qu'il consiste à faire réagir le composé A-CFX-SO₂F avec M^{m+}(OH⁻)ₘ ou 1/mM^{m+}[OSi(CH₃)₃]⁻.

10. Procédé de préparation d'un composé selon la revendication 1 dans lequel Z représente 1/mM^{m+}[-N-SO₂-Q]⁻, 1/mM^{m+}[-CH(SO₂-Q)]⁻ ou 1/mM^{m+}[-C(SO₂-Q)₂]⁻, Q représentant -R_{F} ou -CFX-A, avec X = R_{F} lorsque A = R³, caractérisé en ce qu'il consiste à faire réagir le composé A-CFX-SO₂F avec respectivement 1/mM^{m+}[HNSO₂Q]⁻, 1/mM^{m+}[H(SO₂Q)]⁻ ou 1/mM^{m+}[HC(SO₂Q)₂]⁻, en présence d'une base nucléophile.

11. Procédé de préparation d'un composé selon la revendication 1 présentant une structure symétrique telle que 1/mM^{m+}[(A-CFX-SO₂)₂N]⁻ ou 1/mM^{m+}[(A-CFX-SO₂)₂CH]⁻ ou 1/mM^{m+}[(A-CFX-SO₂)₃C]⁻, caractérisé en ce qu'il consiste à faire réagir le composé A-CFX-SO₂F respectivement avec le nitrure ionique Li₃N ou le carbure ionique C₃Al₄ dans un solvant aprotique polaire.

12. Polymère obtenu par polymérisation d'un compose répondant à la formule générale (1) A-CFX-SO₂Z dans laquelle:
- A représente l'un des groupes R¹R²N-CO-, R³-O-CF₂- ou R³- ;
- Z représente Cl, -OSi(CH₃)₃ ou un groupement ionique différent de O⁻M⁺, M^{m+} représentant un ion d'un métal ayant la valence m, choisi parmi les métaux alcalins, les métaux alcalino-terreux, les métaux de transition et les terres rares, ou un ion ammonium ;
- X représente F, Cl, H ou R_{F} ; X étant R_{F} lorsque A représente R³-;
- les radicaux R¹, R² et R³ identiques ou différents, sont choisis parmi les radicaux organiques non perfluorés polymérisables;
- R_{F} est choisi parmi les radicaux perfluoroalkyle et les radicaux perfluoroaryle.

13. Polymère selon la revendication 12, caractérisé en ce que Z représente un groupement ionique choisi parmi 1/mM^{m+}[-NSO₂Q]⁻, 1/mM^{m+}[-CH(SO₂Q)]⁻ et 1/mM^{m+}[-C(SO₂Q)₂]⁻, Q représentant -R_{F} ou -CFX-A, avec X = R_{F} lorsque A = R³ ; M^{m+} représentant un ion d'un métal ayant la valence m, choisi parmi les métaux alcalins, les métaux alcalino-terreux, les métaux de transition et les terres rares, ou un ion ammonium répondant à la formule NH₍₄₋ⱼ₎Rⱼ⁺, ou un ion amidinium répondant à la formule RC(NH₂₋ⱼRⱼ)₂⁺, ou un ion guanidinium répondant à la formule C(NH₂₋ⱼRⱼ)₃⁺, avec j = 0, 1 ou 2, R étant choisi parmi l'hydrogène, un groupement alkyle, oxaalkyle ou aryle.

14. Polymère selon la revendication 12, caractérisé en ce qu'il est obtenu par copolymérisation avec au moins un autre monomère.

15. Réseau macromoléculaire obtenu par coréticulation d'un monomère selon la revendication 1 avec un polymère préexistant.

16. Matériau à conduction ionique contenant un polymère selon la revendication 15 ou un réseau macromoléculaire selon la revendication 18.

17. Application d'un matériau selon la revendication 16 à la réalisation d'un dispositif électrochimique.

## Claims

1. A compound having the following general formula (1) 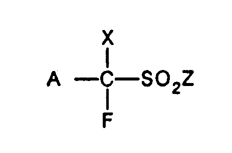 wherein:
A denotes one of the groups: R³-O-CF₂-, R³- or 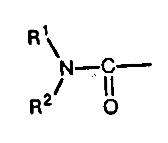 Z denotes F, Cl, -OSi(CH₃)₃ or an tonic grouping, Z being different from F when A denotes R³-O-CF₂- or R³-;
X denotes F, Cl, H or R_{F}, X being R_{F} when A is R³-;
the radicals R¹, R² and R³, which are identical or different, are chosen from among polymerisable non-perfluorinated organic radicals, and
R_{F} is chosen from among perfluoroalkyl radicals and perfluoroaryl radicals.

2. A compound according to claim 1, characterised in that Z denotes an ionic grouping chosen from among 1/mM^{m+}[-O]⁻, 1/mM^{m+}[-NSO₂Q]⁻, 1/mM^{m+}[-CH(SO₂Q)]⁻ and 1/mM^{m+}[-C(SO₂Q)₂]⁻, Q denotes -R_{F} or -CFX-A, with X = R_{F} when A = R³; M^{m+} denotes an ion of a metal having the valency m and chosen from among alkali metals, alkaline earth metals, transition metals and rare earths, or an ammonium ion having the formula NH₍₄₋ⱼ₎Rⱼ⁺, or an amidinium ion having the formula RC(NH₂₋ⱼRⱼ)₂⁺, or a guanidinium ion having the formula C(NH₂₋ⱼRⱼ)₃⁺, with j = 0, 1 or 2, R being chosen from among hydrogen or an alkyl, oxaalkyl or aryl grouping.

3. A compound according to claim 1 or 2, characterised in that R_{F} is chosen from among perfluoroalkyl groupings containing 1 to 8 carbon atoms and perfluoroaryl groupings containing 6 to 8 carbon atoms.

4. A compound according to claim 1, characterised in that the R¹, R² or R³ groupings comprise double bonds such as vinyl, allyl, vinylbenzyl or acryloyl or an oxirane group, an oxetane group, an azetidine group, an aziridine group, an alcohol group, a thiol group, an amine group, an isocyanate group or a trialkoxysilane group.

5. A compound according to claim 1, characterised in that it has one of the following formulae:
1/mM^{m+}[A-CFX-SO₃]⁻,
1/mM^{m+}[A-CFX-SO₂-N-SO₂-R_{F}]⁻,
1/mM^{m+}[A-CFX-SO₂-C(SO₂-R_{F})₂]⁻,
1/mM^{m+}[A-CFX-SO₂-CH(SO₂-R_{F})],⁻.
1/mM^{m+}[A¹-CFX¹-SO₂-N-SO₂-CFX²-A²]⁻,
1/mM^{m+}[A¹-CFX¹-SO₂-CH(SO₂-CFX²-A²)]⁻,
1/mM^{m+}[A¹-CFX¹-SO₂-C(SO₂-CFX²-A²)₂]⁻,
in which A, X and M have the meanings given previously; A¹ and A² are identical or different and chosen from among the previously-mentioned groupings A; X¹ and X² are identical or different and chosen from among the previously-mentioned groupings X, and X is R_{F} if A is R³.

6. A process for the preparation of a compound according to claim 1, in which A denotes R¹R²N-CO- and Z denotes F, characterised in that it consists in reacting sulphonyl acetic acid fluoride F-COCFX-SO₂F with an amine R¹R²NH in the presence of a base.

7. A process for the preparation of a compound according to claim 1, in which Z denotes Cl, characterised in that it consists in reacting the compound A-CFX-SO₂F with a chloride 1/mM^{m+}Cl⁻.

8. A process for the preparation of a compound according to claim 1, in which Z denotes -OSi(CH₃)₃, characterised in that it consists in reacting the compound A-CFX-SO₂F with a silylation agent such as hexamethyl disiloxane.

9. A process for the preparation of a compound according to claim 1, in which Z denotes 1/mM^{m+}[-O]⁻, characterised in that it consists in reacting the compound A-CFX-SO₂F with M^{m+}(OH⁻)ₘ or 1/mM^{m+}[OSi(CH₃)₃]⁻.

10. A process for the preparation of a compound according to claim 1, in which Z denotes 1/mM^{m+}[-N-SO₂-Q]⁻, 1/mM^{m+}[-CH-SO₂-Q]⁻ or 1/mM^{m+}[-C(SO₂-Q)₂]⁻, Q denoting -R_{F} or
-CFX-A, with X = R_{F} when A = R³, characterised in that it consists in reacting the compound A-CFX-SO₂F with 1/mM^{m+}[HNSO₂Q]⁻, 1/mM^{m+}[H(SO₂Q)]⁻ or 1/mM^{m+}[HC(SO₂Q)₂]⁻ respectively, in the presence of a nucleophilic base.

11. A process for the preparation of a compound according to claim 1 having a symmetrical structure such as 1/mM^{m+}[(A-CFX-SO₂)₂N]⁻ or 1/mM^{m+}[(A-CFX-SO₂)₂CH]⁻, or 1/mM^{m+}[(A-CFX-SO₂)₃C]⁻, characterised in that it consists in reacting the compound A-CFX-SO₂F with ionic nitride Li₃N or ionic carbide C₃Al₄ respectively, in a polar aprotic solvent.

12. A polymer obtained by polymerisation of a compound having the general formula (1) A-CFX-SO₂Z in which
A denotes one of the groups: R¹R²N-CO-, R³-O-CF₂- or R³⁻;
Z denotes Cl, -OSi(CH₃)₃ or an ionic grouping different from O⁻M⁺, M^{m+} denoting an ion of a metal having the valency m and chosen from among alkaline metals, alkaline earth metals, transition metals and rare earths, or an ammonium ion;
X denotes F, Cl, H or R_{F}, X being R_{F} when A is R³-;
the radicals R¹, R² and R³, which are identical or different, are chosen from among polymerisable non-perfluorinated organic radicals, and
R_{F} is chosen from among perfluoroalkyl radicals and perfluoroaryl radicals.

13. A polymer according to claim 12, characterised in that Z denotes an ionic grouping chosen from among 1/mM^{m+}[-NSO₂Q]⁻, 1/mM^{m+}[-CH(SO₂Q)]⁻ and 1/mM^{m}_^{+{}[-C(SO₂Q)₂]⁻, Q denoting -R_{F} or -CFX-A, with X = R_{F} when A = R³, M^{m+} denoting an ion of a metal having the valency m and chosen from among alkali metals, alkaline earth metals, transition metals and rare earths, or an ammonium ion having the formula NH₍₄₋ⱼ₎Rⱼ⁺ or an amidinium ion having the formula RC(NH₂₋ⱼRⱼ)₂⁺ or a guanidinium ion having the formula C(NH₂₋ⱼRⱼ)₃⁺ with j = 0, 1 or 2, R being chosen from among hydrogen or an alkyl, oxaalkyl or aryl grouping.

14. A polymer according to claim 12, characterised in that it is obtained by copolymerisation with at least one other monomer.

15. A macromolecular network obtained by co-crosslinking a monomer according to claim 1 with a pre-existing polymer.

16. An ion-conducting material containing a polymer according to claim 15 or a macromolecular network according to claim 18.

17. Use of a material according to claim 16 for making an electrochemical device.

## Patentansprüche

1. Verbindung der nachstehenden allgemeinen Formel (1) 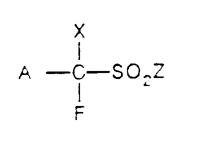 worin:
- A eine der Gruppen R³-O-CF₂-, R³- oder 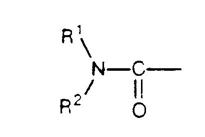 darstellt;
- Z F, Cl, -OSi(CH₃)₃ oder eine ionische Gruppe darstellt, wobei Z nicht F ist, falls A R³-O-CF₂- oder R³ ist;
- X F, Cl, H oder R_{F} darstellt, wobei X R_{F} ist, falls A R³- ist;
- die Reste R¹, R² und R³, gleich oder unterschiedlich, aus organischen, nicht-perfluorierten, polymerisierbaren Resten ausgewählt sind;
- R_{F} aus Perfluoralkyl- und Perfluoraryl-Resten ausgewählt ist.

2. Verbindung nach Anspruch 1, dadurch gekennzeichnet, daß Z eine aus 1/mM^{m+}[-O]⁻, 1/mM^{m+}[-NSO₂Q]⁻, 1/mM^{m+}[-CH(SO₂Q)]⁻ und 1/mM^{m+}[-C(SO₂Q)₂]⁻ ausgewählte ionische Gruppe darstellt, wobei Q -R_{F} oder -CFX-A darstellt, mit X = R_{F}, falls A = R³ ist; wobei M^{m+} ein m-wertiges Metallion, ausgewählt aus Alkalimetallen, Erdalkalimetallen, Übergangsmetallen und Seltenerdmetallen, oder ein Ammoniumion der Formel NH₍₄₋ⱼ₎Rⱼ⁺ oder ein Amidiniumion der Formel RC(NH₂₋ⱼRⱼ)₂⁺ oder ein Guanidiniumion der Formel C(NH₂₋ⱼRⱼ)₃⁺ darstellt, mit j = 0, 1 oder 2, und wobei R aus Wasserstoff, Alkyl-, Oxaalkyl- oder Arylgruppen ausgewählt ist.

3. Verbindung nach irgendeinem der Ansprüche 1 oder 2, dadurch gekennzeichnet, daß R_{F} aus Perfluoralkylgruppen mit 1 - 8 Kohlenstoffatomen und aus Perfluoralkylgruppen mit 6-8 Kohlenstoffatome ausgewählt ist.

4. Verbindung nach Anspruch 1, dadurch gekennzeichnet, daß die Gruppen R¹, R² oder R³ Doppelbindungen vom Vinyl-, Allyl-, Vinylbenzyl- oder Acryloyl-Typ oder eine Oxiran-, Oxethan-, Azetidin-, Aziridin-, Alkohol-, Thiol-, Amino-, Isocyanat- oder Trialkoxysilan-Funktionalität tragen.

5. Verbindung nach Anspruch 1, dadurch gekennzeichnet, daß sie einer der folgenden Formeln entspricht:
1/mM^{m+}[A-CFX-SO₃]⁻,
1/mM^{m+}[A-CFX-SO₂-N-SO₂-R_{F}]⁻,
1/mM^{m+}[A-CFX-SO₂-C(SO₂-R_{F})₂]⁻,
1/mM^{m+}[A-CFX-SO₂-CH(SO₂-R_{F})]⁻,
1/mM^{m+}[A¹-CFX¹-SO₂-N-SO₂-CFX²-A²]⁻,
1/mM^{m+}[A¹-CFX¹-SO₂-CH(SO₂-CFX²-A²)]⁻,
1/mM^{m+}[A¹-CFX¹-SO₂-C(SO₂-CFX²-A²)₂]⁻,
worin A, X und M wie zuvor definiert sind; A¹ und A², gleich oder verschieden, aus den zuvor für A angeführten Gruppen ausgewählt sind; X¹ und X², gleich oder verschieden, aus den zuvor für X angeführten Gruppen ausgewählt sind, wobei X = R_{F} ist, falls A = R³ ist.

6. Verfahren zur Herstellung einer Verbindung nach Anspruch 1, worin A R¹R²N-CO- und Z F darstellt, dadurch gekennzeichnet, daß es aus der Umsetzung von Sulfonylessigsäurefluorid in Gegenwart einer Base mit einem Amin R¹R²NH besteht.

7. Verfahren zur Herstellung einer Verbindung nach Anspruch 1, worin Z Cl darstellt, dadurch gekennzeichnet, daß es aus der Umsetzung der Verbindung A-CFX-SO₂F mit einem Chlorid 1/mM^{m+}Cl⁻besteht.

8. Verfahren zur Herstellung einer Verbindung nach Anspruch 1, worin Z -OSi(CH₃)₃ darstellt, dadurch gekennzeichnet, daß es aus der Umsetzung der Verbindung A-CFX-SO₂F mit einem Silylierungsmittel, wie z.B. Hexamethyldisiloxan, besteht.

9. Verfahren zur Herstellung einer Verbindung nach Anspruch 1, worin Z 1/mM^{m+}[-O]⁻ darstellt, dadurch gekennzeichnet, daß es aus der Umsetzung der Verbindung A-CFX-SO₂F mit M^{m+}(OH⁻)ₘ oder 1/mM^{m+}[OSi(CH₃)₃]⁻ besteht.

10. Verfahren zur Herstellung einer Verbindung nach Anspruch 1, worin Z 1/mM^{m+}[-N-SO₂-Q]⁻, 1/mM^{m+}[-CH(SO₂-Q)]⁻ oder 1/mM^{m+}[-C(SO₂-Q)₂]⁻ darstellt, wobei Q -R_{F} oder -CFX-A darstellt, mit X = R_{F}, falls A = R³ ist, dadurch gekennzeichnet, daß es aus der Umsetzung der Verbindung A-CFX-SO₂F in Gegenwart einer nukleophilen Base mit einem von 1/mM^{m+}[HNSO₂Q]⁻, 1/mM^{m+}[H(SO₂Q)]⁻ oder 1/mM^{m+}[HC(SO₂Q)₂]⁻ besteht.

11. Verfahren zur Herstellung einer Verbindung nach Anspruch 1 mit einer symmetrischen Struktur, wie z.B. 1/mM^{m+}[(A-CFX-SO₂)₂N]⁻ oder 1/mM^{m+}[(A-CFX-SO₂)₂CH]⁻ oder 1/mM^{m+}[(A-CFX-SO₂)₃C]⁻, dadurch gekennzeichnet, daß es aus der Umsetzung der Verbindung A-CFX-SO₂F mit dem ionischen Nitrid Li₃N oder dem ionischen Carbid C₃Al₄ in einem aprotischen, polaren Lösungsmittel besteht.

12. Polymer, erhalten durch Polymerisation einer Verbindung der allgemeinen Formel (1) A-CFX-SO₂Z, worin:
- A eine der Gruppen R¹R²N-CO-, R³-O-CF₂- oder R³- darstellt;
- Z Cl, -OSi(CH₃)₃ oder eine andere ionische Gruppe als O⁻M⁺ darstellt, wobei M^{m+} ein m-wertiges Metallion, ausgewählt aus Alkalimetallen, Erdalkalimetallen, Übergangsmetallen und Seltenerdmetallen, oder ein Ammoniumion darstellt;
- X F, Cl, H oder R_{F} darstellt, wobei X R_{F} ist, falls A R³- ist;
- die Reste R¹, R² und R³, gleich oder unterschiedlich, aus organischen, nicht-perfluorierten, polymerisierbaren Resten ausgewählt sind;
- R_{F} aus Perfluoralkyl- und Perfluoraryl-Resten ausgewählt ist.

13. Polymer nach Anspruch 12, dadurch gekennzeichnet, daß Z eine aus 1/mM^{m+}[-NSO₂Q]⁻, 1/mM^{m+}[-CH(SO₂Q)]⁻ und 1/mM^{m+}[-C(SO₂Q)₂]⁻ ausgewählte ionische Gruppe darstellt, wobei Q -R_{F} oder -CFX-A darstellt, mit X = R_{F}, falls A = R³ ist; wobei M^{m+} ein m-wertiges Metallion, ausgewählt aus Alkalimetallen, Erdalkalimetallen, Übergangsmetallen und Seltenerdmetallen, oder ein Ammoniumion der Formel NH₍₄₋ⱼ₎Rⱼ⁺ oder ein Amidiniumion der Formel RC(NH₂₋ⱼRⱼ)₂⁺ oder ein Guanidiniumion der Formel C(NH₂₋ⱼRⱼ)₃⁺ darstellt, mit j = 0, 1 oder 2, und wobei R aus Wasserstoff, Alkyl-, Oxaalkyl- oder Arylgruppen ausgewählt ist.

14. Polymer nach Anspruch 12, dadurch gekennzeichnet, daß es durch Copolymerisation mit zumindest einem anderen Monomer erhalten wird.

15. Makromolekulares Netzwerk, das durch Covernetzung eines Monomers nach Anspruch 1 mit einem zuvor gebildeten Polymer erhalten wird.

16. Ionenleitendes Material, das ein Polymer nach Anspruch 12 oder ein makromolekulares Netzwerk nach Anspruch 15 enthält.

17. Verwendung eines Materials nach Anspruch 16 zur Herstellung einer elektrochemischen Vorrichtung.
